# EUROPEAN PATENT APPLICATION

(11) **EP 3 936 773 A1**
(43) Date of publication of application: **12.01.2022**
(21) Application number: 20810400.0
(22) Date of filing: 12.05.2020
(51) Int. Cl.: F24F 6/00, F24F 7/00, F24F 7/007, F24F 11/80

(54) **CONTROL SYSTEM, SENSATION PRESENTATION DEVICE, AND METHOD FOR CONTROLLING SENSATION PRESENTATION**

(30) Priority: 17.05.2019 JP 2019093982
(71) Applicant: Kabushiki Kaisha Tokai Rika Denki Seisakusho, Aichi 480-0195 (JP)
(72) Inventor: TAKAI, Toshihito, Niwa-gun, Aichi 480-0195 (JP); SASAKI, Shinobu, Niwa-gun, Aichi 480-0195 (JP); IWATA, Kenji, Niwa-gun, Aichi 480-0195 (JP); TAKAGI, Yuka, Niwa-gun, Aichi 480-0195 (JP); HIROSE, Fumiaki, Niwa-gun, Aichi 480-0195 (JP); OHNISHI, Takeshi, Niwa-gun, Aichi 480-0195 (JP)
(74) Representative: TBK
(86) International application number: PCT/JP2020/018895
(87) International publication number: WO 2020/235384

(57) **Abstract**

A state of a subject is led to a target state more reliably. A control system includes a sensation presentation unit that performs sensation presentation to a subject, and a control unit that causes the sensation presentation unit to perform sensation presentation corresponding to a target state that is a state desired to be realized in the subject.

## Description

### Technical Field

The present invention relates to a control system, a sensation presentation device, and a sensation presentation control method.

### Background Art

In recent years, products for giving a relaxing effect to a subject have become widespread. For example, the following Patent Literature 1 discloses an aroma set for giving a relaxing effect to a subject by diffusing a scent into the air.

### Citation List

### Patent Literature

Patent Literature 1: JP 2017-078529A

### Summary of Invention

### Technical Problem

However, products of the related art merely perform a predetermined monotonous operation, and it may be difficult to cause a state of a target to transition to a target state that is desired to be realized.

Therefore, the present invention has been made in light of the above problem, and an object of the present invention is to provide a control system, a sensation presentation device, and a sensation presentation control method that are novel and improved, capable of more reliably leading a state of a subject to a target state.

### Solution to Problem

According to an aspect of the present invention, there is provided a control system comprising a sensation presentation unit that performs sensation presentation to a subject; and a control unit that causes the sensation presentation unit to perform sensation presentation corresponding to a target state that is a state desired to be realized in the subject.

The control unit may cause the sensation presentation unit to start sensation presentation corresponding to a first target state, and then changes the sensation presentation performed by the sensation presentation unit to sensation presentation corresponding to a second target state different from the first target state.

The control unit may control a change of sensation presentation performed by the sensation presentation unit such that transition to the second target state is started or realized at a set time point.

The first target state may be a state in which a degree of alertness of the subject is lower than in the second target state.

The control system may further comprise a detection unit that detects a state of the subject. The control unit may cause the sensation presentation unit to perform the sensation presentation corresponding to the target state on the basis of a state of the subject detected by the detection unit being a predetermined state.

According to another aspect of the present invention, there is provided a sensation presentation device that performs sensation presentation corresponding to a target state on the basis of a control signal that is input according to the target state desired to be realized in a subject.

According to another aspect of the present invention, there is provided a sensation presentation control method comprising: specifying a target state that is a state desired to be realized in a subject; and causing a sensation presentation unit to perform sensation presentation corresponding to the target state.

### Advantageous Effects of Invention

According to the present invention described above, it is possible to more reliably lead a state of a subject to a target state.

### Brief Description of Drawings

[FIG. 1] FIG. 1 is an explanatory diagram illustrating a configuration of a control system 10 according to an embodiment of the present invention.
[FIG. 2] FIG. 2 is a flowchart illustrating an example A of control of sensation presentation.
[FIG. 3] FIG. 3 is a flowchart illustrating an example B of control of sensation presentation.
[FIG. 4] FIG. 4 is a flowchart illustrating an example C of control of sensation presentation.

### Description of Embodiments

Hereinafter, embodiments of the present invention will be described in detail with reference to the accompanying drawings. In the specification and the drawings, constituents having substantially the same functional configuration are given the same reference numerals, and thus repeated description will be omitted.

### <1. Outline>

First, an outline of an embodiment of the present invention will be described. In recent years, aroma diffusers that diffuse scents into the air have become widespread. According to such an aroma diffuser, a subject can be brought into a relaxed state by giving a pleasant stimulus to the sense of smell among the five human senses.

However, a state (hereinafter, also referred to as a target state) that is desired to be realized in a subject is not necessarily one type. For example, in a case where a subject takes a nap, an initial target state of the subject is a sleeping state, but then a target state of the subject may change to a wakeful state. Thus, it may be difficult to bring the subject into a desired state in a product of the related art that performs a predetermined monotonous operation.

The inventor of the present invention has created an embodiment of the present invention with the above circumstances as the first point of view. A control system according to the embodiment of the present invention includes a sensation presentation unit that performs sensation presentation to a subject and a control unit that causes the sensation presentation unit to perform sensation presentation corresponding to a target state that is a state desired to be realized in the subject.

In the present specification, a user (a driver, a passenger, or the like) of a moving object will be mainly described as the above subject. However, the subject is not limited to a user of a moving object, but may be another person with a target state (for example, a person who desires to transition to a sleeping state, a person who desires to transition to a state of concentrating on study or work, or a person who desires transition to a wakeful state). The subject is not limited to a human, and may be an animal such as a dog, a cat, a horse, or a monkey.

In the present specification, a real vehicle will be mainly described as the above moving object. However, a concept of the moving object is not limited to a real vehicle. That is, the concept of the moving object includes real or virtual vehicles, aircraft, or ships. More specifically, examples of the moving object include real vehicles (for example, an automobile, a bus, a bike, a locomotive, or a train), aircraft, (for example, an airplane, a helicopter, a glider, or an airship), and ships (for example, a passenger ship, a cargo ship, or a submarine). The moving object may be a virtual vehicle, aircraft, or ship, for example, a simulator or a game machine related to a vehicle, an aircraft, or a ship. The moving object may be not only a manned object but also an unmanned object. For example, the moving object may be an unmanned transport vehicle, an unmanned aircraft, an unmanned ship, or a radio-controlled object that is operated remotely by a subject.

The above sensation presentation is presentation of a stimulus that acts on at least one of the five senses of the subject. That is, examples of the sensation presentation includes tactile presentation that is presentation of a stimulus that acts on the sense of touch, visual presentation that is presentation of a stimulus that acts on the sense of sight, olfactory presentation that is presentation of a stimulus that acts on the sense of smell, and gustatory presentation that is presentation of a stimulus that acts on the sense of taste, and auditory presentation that is presentation of a stimulus that acts on the sense of hearing.

Hereinafter, a configuration and control examples of a control system according to an embodiment of the present invention will be described in detail in order.

### <2. Configuration of control system>

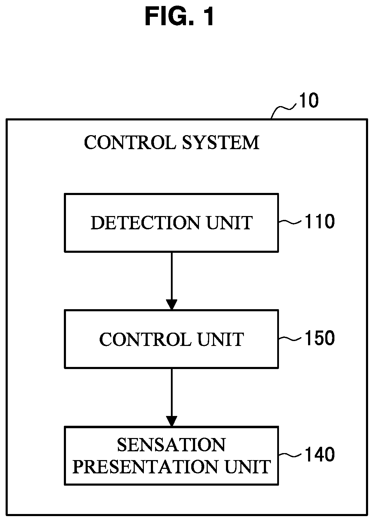

FIG. 1 is an explanatory diagram illustrating a configuration of a control system 10 according to an embodiment of the present invention. As illustrated in FIG. 1, the control system 10 according to the embodiment of the present invention includes a detection unit 110, a sensation presentation unit 140, and a control unit 150.

### (Detection unit 110)

The detection unit 110 is, for example, a sensor that continuously detects various states of a subject. The detection unit 110 may be a camera that images the subject's face, or an optical sensor, a temperature sensor, or a pulse sensor that detects the heartbeat or a skin temperature of the subject, or may be a combination thereof. The detection unit 110 may be an operation detection unit such as a button, a switch, a touch panel, or a lever for detecting an operation.

### (Sensation presentation unit 140)

The sensation presentation unit 140 performs one of the above-described tactile presentation, visual presentation, olfactory presentation, gustatory presentation, and auditory presentation, or a combination of two or more thereof. Examples of configurations for tactile presentation include a configuration in which tactile presentation is performed by using a vibration stimulus, a configuration in which tactile presentation is performed by using electrical stimulus, a configuration in which tactile presentation is performed by using a temperature change, a configuration in which tactile presentation related to a force sensation (for example, presentation of a sensation of being pushed by an object, presentation of a sensation of coming into contact with an object, or presentation of a sensation of tightening) is performed, or a configuration in which tactile presentation related to a skin sensation (for example, presentation of a rough sensation or presentation of a slippery sensation) is performed.

Examples of configurations for visual presentation include a configuration having a lighting function and a configuration capable of changing the light transmittance such as dimming glass. Examples of a configuration for olfactory presentation includes a configuration in which a scent is diffused in the air. Examples of configurations for gustatory presentation include a configuration for adjusting a taste of a substance to be placed in the oral cavity, and a configuration for adjusting a taste of a substance already placed in the oral cavity. Examples of configurations for auditory presentation include a configuration for generating air vibration, such as a speaker or an earphone, and a configuration having a bone conduction function.

### (Control unit 150)

The control unit 150 causes the sensation presentation unit 140 to perform sensation presentation corresponding to a target state of a subject. For example, the control unit 150 may specify a target state of the subject on the basis of a result of detection in the detection unit 110, and may cause the sensation presentation unit 140 to perform sensation presentation corresponding to the specified target state. The functions of the control unit 150 may be realized in cooperation with, for example, a processor such as a central processing unit (CPU) or a micro controller unit (MCU), software, and a storage medium such as a read only memory (ROM) or a random access memory (RAM). Hereinafter, some examples of control of sensation presentation will be described.

### <3. Examples of control of sensation presentation>

### (Control example A)

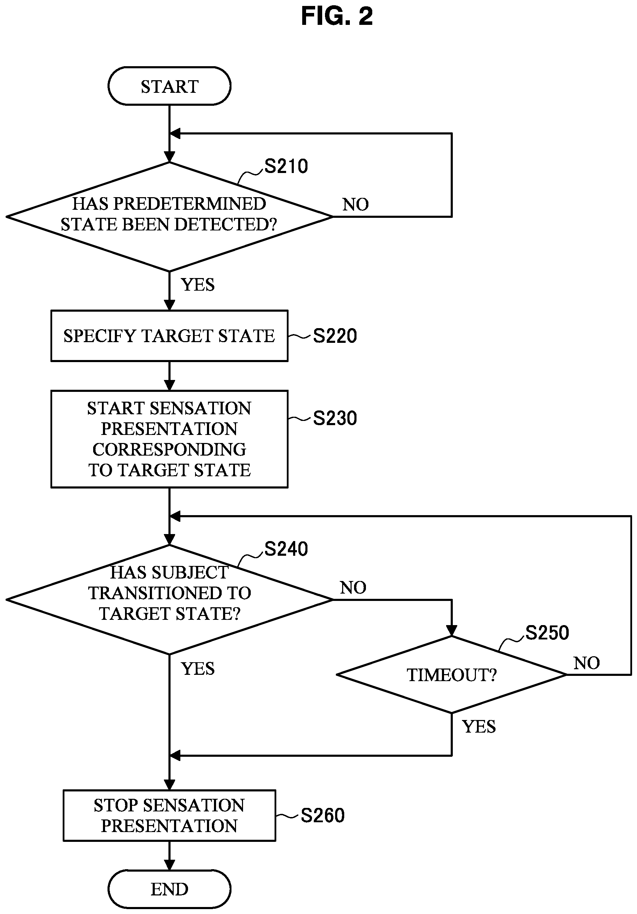

As will be described with reference to FIG. 2, a control example A involves control in which sensation presentation to a subject is started to be performed in a case where a state of the subject becomes a predetermined state.

FIG. 2 is a flowchart illustrating the example A of control of sensation presentation. As illustrated in FIG. 2, first, in a case where it has been detected that a state of a subject is a predetermined state by the detection unit 110 (S210/Yes), the control unit 150 specifies a target state of the subject (S220). The control unit 150 causes the sensation presentation unit 140 to start sensation presentation corresponding to the target state (S230).

Thereafter, the control unit 150 determines whether or not a state of the subject has transitioned to the target state (S240). In a case where a state of the subject has not transitioned to the target state (S240/No), the sensation presentation corresponding to the target state is continuously performed before a timeout occurs (S250/No), and the determination in S240 is repeatedly performed. Here, the timeout may mean that a predetermined time elapses after the predetermined state is detected in S210, or a predetermined time elapses after the sensation presentation corresponding to the target state is started in S230.

On the other hand, in a case where a state of the subject has transitioned to the target state (S240/Yes) or a timeout has occurred (S250/Yes), the control unit 150 causes the sensation presentation unit 140 to stop the sensation presentation on the subject (S260).

### - Specific application example of control example A

The control example A may be applied to, for example, a case where an infant is sitting in a car seat in a rear seat of a vehicle and a parent is driving the vehicle. If the infant begins to cry while the parent is driving the vehicle, it is difficult for the parent to take care of the infant. Therefore, it is considered that the function of the control system 10 is installed in the car seat, and the car seat executes the control example A to bring the infant into a resting state. In this case, the subject may be the infant, the predetermined state may be the state in which the infant is crying, and the target state may be the resting state. The resting state is a state in which the infant is not crying and is calm.

The sensation presentation corresponding to the resting state, that is, the sensation presentation for bringing the infant into the resting state that is the target state may be a pseudo sensation presentation simulating the infant being taken care of by its mother. For example, in a case where a heating portion such as a heater is provided as an element of the sensation presentation unit 140, the control unit 150 may increase a temperature of the heating portion to the mother's temperature (such as the mother's temperature measured in advance, the mother's real-time temperature, or the mother's general temperature) such that a sensation of the infant being held by the mother is presented. In a case where a speaker is provided near the infant's ear as an element of the sensation presentation unit 140, the control unit 150 may cause the speaker to output the voice of the mother. In a case where a power portion for rocking the car seat is provided as one element of the sensation presentation unit 140, the control unit 150 may cause the power portion to rock the car seat. In a case where a vibrator is provided as one element of the sensation presentation unit 140, the control unit 150 may cause the vibrator to output vibration expressing the heartbeat of the mother. In a case where a steering wheel is provided with a sensor for detecting an operation such as tapping, the control unit 150 may vibrate the vibrator provided in the car seat at a timing when the mother taps the steering wheel.

The sensation presentation for bringing the infant into the resting state that is the target state may be sensation presentation that is comforting to the infant. For example, when a vibrator is provided in the car seat as an element of the sensation presentation unit 140, the control unit 150 may output a comforting vibration to the vibrator. In a case where a sound output portion such as a speaker or a bone conduction portion is provided as one element of the sensation presentation unit 140, the control unit 150 may cause the sound output portion to output white noise. In a case where a wind generation portion such as a fan is provided as one element of the sensation presentation unit 140, the control unit 150 may cause the wind generation portion to blow wind toward a diaper so as to reduce the stuffiness of the diaper. In a case where the infant's face is warm, the control unit 150 may cause the wind generation portion to blow the wind toward the infant's face.

With such a configuration, it is possible to bring the infant to the resting state even in a case where it is difficult for the parent to take care of the infant.

The control unit 150 may control the sensation presentation for feeding back a state of the infant to the parent driving the vehicle. For example, the control unit 150 may cause the vibrator provided on the steering wheel to output vibration expressing the heartbeat of the infant. In a case where the control unit 150 determines that a state of the infant is abnormal from the infant's heartbeat, skin temperature, or the like, the control unit 150 may vibrate the vibrator provided on the steering wheel.

In the above description, the example in which the control example A is applied to the case where the infant is sitting in the car seat of the rear seat of the vehicle and the parent is driving the vehicle has been described, but the control example A may also be applied to other cases. For example, the control example A may be applied to various cases where a parent cannot use his/her hands, such as a case where an infant is sleeping in a crib at home and a parent is cooking in a kitchen.

### (Control example B)

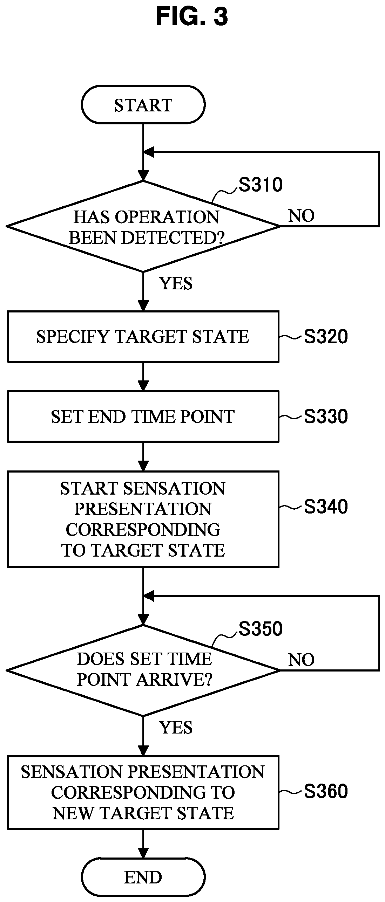

As will be described with reference to FIG. 3, a control example B involves control in which sensation presentation is changed according to a change of a target state.

FIG. 3 is a flowchart illustrating the example B of control of sensation presentation. As illustrated in FIG. 3, first, when an operation by a subject or another person has been detected (S310/Yes), the control unit 150 specifies a target state of the subject (S320) and sets a time point (a set time point in the following description of the control example B) at which sensation presentation corresponding to the target state is finished (S330).

Thereafter, the control unit 150 causes the sensation presentation unit 140 to start the sensation presentation corresponding to the target state (S340). The control unit 150 causes the sensation presentation unit 140 to continue the sensation presentation corresponding to the target state until the set time point arrives (S350/No), and when the set time point arrives (S350/Yes), causes the sensation presentation unit 140 to perform sensation presentation corresponding to a new target state different from the initial target state (S360).

### - Specific application example of control example B

The control example B may be applied to a case where a vehicle user such as a taxi driver or a driver who drives a vehicle in business takes a nap in the rear seat. In this case, it is desirable that the vehicle user has a good quality and efficient sleep in a short time such as 15 minutes and then awakens fully in order to eliminate sleep deprivation or reduce fatigue. Thus, installing the function of the control system 10 in the rear seat of the vehicle for the rear seat of the vehicle to execute the control example B and allow a vehicle to have good quality and efficient sleep and a smooth transition between a sleeping state and a wakeful state is considered. In this case, the subject is the vehicle user, the initial target state (first target state) is the sleeping state, and the new target state (second target state) is the wakeful state. An operation for specifying the target state may be an operation of a predetermined button or switch by the vehicle user, and the set time point may be a time point designated by the vehicle user, and a time point after a predetermined time (for example, 15 minutes) elapses, or a time point after a time designated by the vehicle user elapses.

Sensation presentation corresponding to the sleeping state, that is, sensation presentation for bringing the vehicle user into the sleeping state may be presentation of a sensation for relaxing the vehicle user. For example, in a case where a configuration capable of changing the light transmittance, such as dimming glass, is provided as one element of the sensation presentation unit 140, the control unit 150 may reduce the light transmittance of the configuration to reduce the brightness of the rear seat. In a case where a sound output portion such as a speaker or a bone conduction portion is provided as one element of the sensation presentation unit 140, the control unit 150 may cause the sound output portion to output music having a relaxing effect at a low volume. In a case where a configuration for diffusing a scent into the air is provided as one element of the sensation presentation unit 140, the control unit 150 may cause the configuration to output a pleasant scented vapor. In a case where a movable rear seat is provided as one element of the sensation presentation unit 140, the control unit 150 may cause the rear seat to flatten the seat surface or to put a footrest out from an non-use position to a use position. In a case where a heat generation portion is provided as one element of the sensation presentation unit 140, the control unit 150 may cause the heat generation portion to generate heat.

Sensation presentation corresponding to the wakeful state that is the new target state, that is, sensation presentation for bringing the vehicle user into the wakeful state that is the new target state may be different from or opposite to sensation presentation for bringing the vehicle user to the sleeping state. For example, when a configuration capable of changing the light transmittance, such as dimming glass is provided, is provided as one element of the sensation presentation unit 140, the control unit 150 may increase the light transmittance of the configuration to increase the brightness of the rear seat. In a case where a configuration having a lighting function is provided as one element of the sensation presentation unit 140, the control unit 150 may cause the configuration to emit light or increase a light emission intensity. In a case where a sound output portion such as a speaker or a bone conduction portion is provided as one element of the sensation presentation unit 140, the control unit 150 may increase an output volume of the sound output portion or output an alarm sound. In a case where a movable rear seat is provided as one element of the sensation presentation unit 140, the control unit 150 may make the rear seat have unevenness on the seat surface, or return a footrest to an non-use position. In a case where a heat generation portion is provided as one element of the sensation presentation unit 140, the control unit 150 may increase an amount of heat generated by the heat generation portion. In a case where a vibrator is provided as one element of the sensation presentation unit 140, the control unit 150 may vibrate the vibrator.

With such a configuration, it is possible to realize state transition of a user between a plurality of target states, and, for example, a user of a vehicle can have a good quality and efficient sleep in a short time and then transition to a wakeful state.

### - Modification example

In the above description, a taxi driver or a driver who drives a vehicle in business has been described as a vehicle user, but the vehicle user is not limited to the driver. For example, in a share car service that has become widespread in recent years, some users use a share car not for the purpose of moving but for the purpose of taking a rest or a nap by obtaining space in the car, and the control example B may be applied to these users. Here, it is assumed that sensation presentation that easily brings into a sleeping state and sensation presentation that easily brings a user into a wakeful state are not a little different depending on users. Thus, the control unit 150 may customize sensation presentation for bringing a user into a sleeping state and sensation presentation for bringing the user into a wakeful state by using profile information of the user of a share car. With such a configuration, it is possible to further improve a satisfaction level of a vehicle user. The application to a vehicle for business purposes such as a taxi and a share car has been mainly described hitherto, but the control example B can be similarly applied to a normal vehicle for non-business purposes. In that case, it may also be possible to customize sensation presentation by using owner information registered in advance.

The example in which the vehicle user sleeps in the rear seat of the vehicle has been described above, but the control example B may also be applied to a case where the vehicle user stops the vehicle on a road shoulder and sleeps in the driver's seat. Even in a case where the rear seat or the driver's seat is used, the hardness of the seat suitable for sleeping and the hardness of the seat suitable for other purposes (for example, movement) are considered to be different. Thus, in a case where a configuration for changing the absolute hardness of the seat or the sensory hardness is provided as one element of the sensation presentation unit 140, the control unit 150 may cause to the configuration to change the hardness of the seat according to a target state. The absolute hardness may be controlled by using, for example, an amount of air contained in the seat, and the sensory hardness may be controlled by using a vibration pattern of the seat. The hardness of the seat according to the target state may be set by default, or may be customized by the vehicle user. The control unit 150 may specify a sleeping state as a target state in a case where an operation of setting a reclining amount of the seat to a threshold value or more has been detected, and control the hardness of the seat to a hardness suitable for the sleeping state.

In the above description, the example in which the control example B is applied to a vehicle has been described, but the control example B may also be applied to other cases. For example, the control example B may be applied to various cases where an initial target state is a sleeping state and then a target state changes to a wakeful state, such as a case where a user uses a bed at home. The control example B may be applied to various industries such as transportation industries such as railroad and aircraft, maintenance and inspection industries, a security industry, a medical industry, and, more specifically, the control example B may be applied to equipment for personnel to take a nap in a facility where personnel in these industries take a nap.

In the above description, the example in which an initial target state is a sleeping state and a new target state is a wakeful state has been described, but the target state is not limited to such an example. For example, one of the initial target state or the new target state may be a concentrated state, and the other of the initial target state or the new target state may be a relaxed state. Similarly, the control example B may be applied to transition between various other states.

### (Control example C)

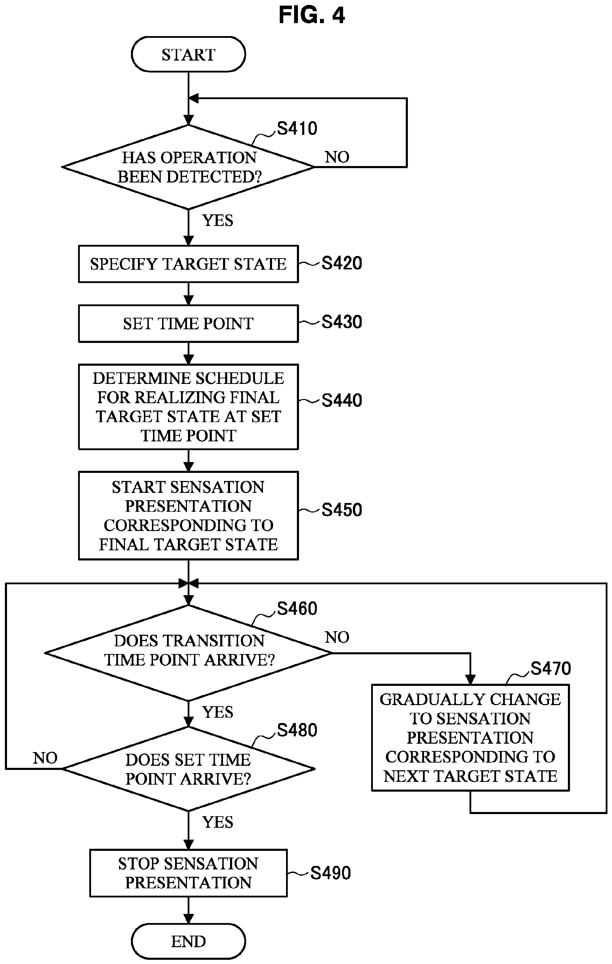

As will be described with reference to FIG. 4, a control example C involves control in which sensation presentation is gradually changed such that a target state is realized at a set time point.

FIG. 4 is a flowchart illustrating the example C of control of sensation presentation. As illustrated in FIG. 4, first, when an operation by a subject or another person has been detected (S410/Yes), the control unit 150 specifies one or more target states of the subject (S420), and sets a time point (a set time point in the following description of the control example C) at which a final target state is desired to be realized (S430). The control unit 150 determines a transition schedule for sensation presentation for realizing the final target state at the set time point (S440).

Subsequently, the control unit 150 causes the sensation presentation unit 140 to start sensation presentation corresponding to the initial target state (S450). Each time a transition time point according to the transition schedule arrives (S460/Yes), the control unit 150 gradually changes sensation presentation performed by the sensation presentation unit 140 to sensation presentation corresponding to the next target state (S470).

Thereafter, when the set time point arrives (S480/Yes), the control unit 150 causes the sensation presentation unit 140 to finish the sensation presentation (S490).

### - Specific application example of control example C

Similarly to the control example B, the control example C may be applied to a case where a vehicle user such as a taxi driver or a driver who drives a vehicle in business takes a nap. In this case, the subject is the vehicle user, and the one or more target states include a sleeping state, a good sleep continuation state, and a wakeful state. An operation for specifying the target state may be an operation of a predetermined button or switch by the vehicle user, and the set time point is a time point designated by the vehicle user, and a time point after a predetermined time (for example, 15 minutes) elapses, or a time point after a time designated by the vehicle user elapses.

Specific examples of sensation presentation corresponding to the sleeping state and sensation presentation corresponding to the wakeful state are as described in the control example B. The good sleep continuation state is a state in which a user is continuously in a good sleeping state. In a case where a vibrator is provided as one element of the sensation presentation unit 140, the sensation presentation unit 140 may first generate vibration to realize a comfortable and early sleeping state as the sensation presentation corresponding to the sleeping state, and, when the transition time point arrives, gradually change a vibration pattern to a vibration pattern corresponding to the good sleep continuation state, and further, when the transition time point arrives, gradually change the vibration pattern to a vibration pattern corresponding to the wakeful state.

With such a configuration, it is possible to realize a smooth state transition of a user between a plurality of target states, and, for example, the user can have good quality sleep in a short time and then smoothly transition to the wakeful state. Each modification example described in the control example B may also be applied to the control example C. That is, the control example C may also be applied to a normal vehicle for non-business purposes and a facility for personnel in various industries to take a nap.

### <4. Conclusion>

As described above, according to the embodiment of the present invention, it is possible to more reliably lead a state of a subject such as a vehicle user to a target state.

Although the preferred embodiment of the present invention has been described in detail with reference to the accompanying drawings, the present invention is not limited to such examples. It is clear that a person skilled in the art can conceive of various changes or modifications within the scope of the technical ideas described in the claims, and these are also naturally understood to belong to the technical scope of the present invention.

The respective steps in the process in the control system 10 of the present specification do not necessarily have to be processed in chronological order in the order described as the flowchart. For example, the respective steps in the process in the control system 10 may be processed in an order different from the order described in the flowchart, or may be processed in parallel.

The respective constituents of the control system 10 may be integrally disposed in one device, or may be separately disposed in two or more devices. For example, a control device having the function of the control unit 150 and a sensation presentation device having the function of the sensation presentation unit 140 may be separately configured, and a control signal may be output from the control device to the sensation presentation device in a wired or wirelessly manner such that the functions and the operations of the control system 10 are realized.

A computer program for causing the hardware such as the CPU, the ROM, and the RAM built in the control system 10 to realize the same function as that of each constituent of the control system 10 described above may be created. A storage medium storing the computer program is also provided.

### Reference Signs List

- 10: control system
- 110: detection unit
- 140: sensation presentation unit
- 150: control unit

## Claims

1. A control system comprising:
a sensation presentation unit that performs sensation presentation to a subject; and
a control unit that causes the sensation presentation unit to perform sensation presentation corresponding to a target state that is a state desired to be realized in the subject.

2. The control system according to claim 1,
wherein the control unit causes the sensation presentation unit to start sensation presentation corresponding to a first target state, and then changes the sensation presentation performed by the sensation presentation unit to sensation presentation corresponding to a second target state different from the first target state.

3. The control system according to claim 2,
wherein the control unit controls a change of sensation presentation performed by the sensation presentation unit such that transition to the second target state is started or realized at a set time point.

4. The control system according to claim 2 or 3,
wherein the first target state is a state in which a degree of alertness of the subject is lower than in the second target state.

5. The control system according to any one of claims 1 to 4, further comprising:
a detection unit that detects a state of the subject,
wherein the control unit causes the sensation presentation unit to perform the sensation presentation corresponding to the target state on the basis of a state of the subject detected by the detection unit being a predetermined state.

6. A sensation presentation device that performs sensation presentation corresponding to a target state on the basis of a control signal that is input according to the target state desired to be realized in a subject.

7. A sensation presentation control method comprising:
specifying a target state that is a state desired to be realized in a subject; and
causing a sensation presentation unit to perform sensation presentation corresponding to the target state.
